(19) Europäisches Patentamt
European Patent Office
Office européen des brevets

(11) Publication number: **0 165 637**
**B1**

(12) # EUROPEAN PATENT SPECIFICATION

(45) Date of publication of patent specification: **27.07.88**

(51) Int. Cl.$^4$: **C 07 D 205/04**

(21) Application number: **85200882.0**

(22) Date of filing: **04.06.85**

(54) **Azetidine derivatives.**

(30) Priority: **19.06.84 GB 8415614**

(43) Date of publication of application:
**27.12.85 Bulletin 85/52**

(45) Publication of the grant of the patent:
**27.07.88 Bulletin 88/30**

(84) Designated Contracting States:
**AT BE CH DE FR GB IT LI LU NL SE**

(56) References cited:
**EP-A-0 001 280**
**EP-A-0 114 079**

**The file contains technical information submitted after the application was filed and not included in this specification**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **De Nie-Sarink, Margaretha Johanna**
**Badhuisweg 3**
**NL-1031 CM Amsterdam (NL)**
Inventor: **Scholes, Gary**
**Carel van Bylandtlaan 30**
**NL-2596 HR The Hague (NL)**

(74) Representative: **Hunter, Keith Roger Ian et al**
**4 York Road**
**London SE1 7NA (GB)**

EP 0 165 637 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to azetidine derivatives and their use as intermediates in the preparation of azetidine-3-carboxylic acid and salts thereof.

European Patent Application Publication No. 29265 discloses that 3-carboxyazetidine (azetidine-3-carboxylic acid) and related compounds are chemical hybridising agents, their mode of action presumably being based on their ability to produce male sterility in plants. That application also describes a process for their preparation, starting from 3-cyano-1-diphenylmethylazetidine, which may be prepared by methods known *per se.*

According to the present invention there are provided azetidine derivatives of formula

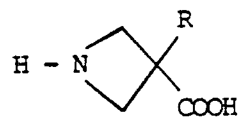

(I)

and salts thereof, wherein R is a group $—CH_2OH$ or —COOH.

The invention also comprises the use of an azetidine derivative of formula I or a salt thereof as an intermediate in the preparation of azetidine-3-carboxylic acid or a salt thereof. Such preparation may conveniently be carried out by heating a compound of formula I wherein R is a group —COOH or a salt thereof in acidic medium at a temperature of at least 60°C. In this preparation the compound of formula I wherein R is a group —COOH may, if desired, be generated by oxidation of a compound of formula I wherein R is a group $—CH_2OH$.

The compound of formula I wherein R is a group —COOH is insoluble in aqueous acidic medium, whereas azetidine-3-carboxylic acid is soluble. Completion of the reaction may thus readily be determined visually, when a clear solution is obtained.

The acid medium may comprise a mineral acid, such as nitric, hydrohalic (e.g. hydrochloric) or sulphuric acid, or a carboxylic acid, preferably formic or acetic acid.

In the case of a carboxylic acid, the concentration of the acid is preferably at least 50% v/v, and is advantageously at least 70 v/v (in the case of formic acid preferably about 80% v/v). When acetic acid is employed it may conveniently be used in the form of glacial acetic acid.

The compound of formula I is preferably heated in the acidic medium at a temperature of at least 80°C. Conveniently it is heated at the reflux temperature of the reaction mixture.

Oxidation of a compound of formula I wherein R is a group $—CH_2OH$ to the corresponding compound wherein R is —COOH may conveniently be effected by treatment with nitric acid followed by passage of oxygen into the reaction mixture, or by catalytic oxidation using oxygen over a catalyst such as platinum or palladium on carbon.

Azetidine derivatives of formula I may conveniently be prepared from compounds of formula

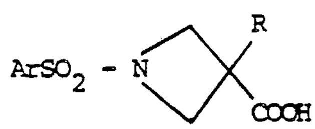

(II)

where R is $—CH_2OH$ or —COOH and Ar is an optionally substituted aryl group, by removal of the $ArSO_2$ group, for example using sodium in liquid ammonia.

Preferably Ar represents an aryl group containing one aromatic nucleus and one comparatively small substituent, e.g. a $C_{1-4}$ alkyl group, an amino group or an alkyl-substituted amino group, which substituent preferably is located at the position para to the $—SO_2—$ moiety. Ar conveniently represents a para-tolyl group.

The compounds of formula II and processes for their preparation, are disclosed in the European Patent Application Publication No. 114,079.

Azetidine derivatives of formula I wherein R is —COOH may alternatively be prepared by treating a compound of formula

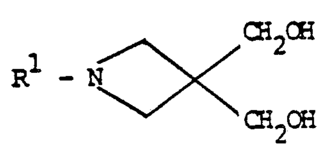

(III)

wherein $R^1$ represents a hydrogen atom or a group of formula $R^2R^3CH—$, wherein $R^2$ and $R^3$ are independently selected from hydrogen, alkyl (preferably $C_{1-6}$ alkyl) and optionally substituted aryl (preferably $C_{6-12}$ aryl) moieties with nitric acid and subsequently passing oxygen into the reaction mixture.

Optional substituents in an aryl moiety $R^2$ and/or $R^3$ include one or more substituents selected from the group consisting of $C_{1-4}$alkyl, $C_{1-4}$alkoxy, halo, nitro and ($C_{1-6}$alkoxy)carbonyl moieties.

Preferably at least one of $R^2$ and $R^3$ in the group $R^2R^3CH$— is hydrogen. Conveniently R in formula III represents a hydrogen atom or a group of formula $R^2CH_2$— wherein $R^2$ represents hydrogen, a $C_{1-3}$ alkyl group or a phenyl group.

The compound of formula III may conveniently be treated with nitric acid at a temperature in the range 35°C to the reflux temperature of the reaction mixture, conveniently a temperature in the range 40 to 85°C. Temperatures in the range of 45 to 60°C are very effective.

It is preferred that the concentration of nitric acid used in the treatment of the compound of formula III be at least 40% w/w. Conveniently the concentration of the nitric acid used is in the range 50 to 65% w/w.

Passage of oxygen into reaction mixtures described above is conveniently achieved mixtures by bubbling air therethrough, preferably at a temperature in the range 90°C to reflux temperature.

Compounds of formula III are described, together with methods for their preparation, in UK Patent Specification No. 1,169,027, and their treatment with nitric acid is described and claimed in European Patent Application Publication No. 165636.

The invention will be further understood from the following examples.

Example 1

Preparation of 3-hydroxymethylazetidine-3-carboxylic acid

1-Tosyl-3-hydroxymethylazetidine-3-carboxylic acid (2.6 g, 0.0096 mol) (prepared as in Example 9A of European Patent Application Publication No. 114,079) was dissolved in liquid ammonia (100 ml) at −50°C. To the resulting solution small pieces of metallic sodium were added until the blue colour persisted for 15 mins. At this stage 1.25 g (0.0544 g atom) sodium had been added. Solid ammonium chloride was then added to disperse the blue colour and the ammonia was allowed to evaporate under a stream of nitrogen. Water was added to the residue and the solution acidified with 10% (w/w) HCl to pH 4. The solution was filtered and then passed over a column containing Dowex 50W-X8 ($H^+$) (Registered Trade Mark) ionexchange resin (100 ml). Eluting with water until neutral gave an acidic fraction which was not analysed further. Elution with 2N $NH_4OH$ gave, after evaporation to dryness, an off-white solid (0.8 g) which was recrystallised from water/methanol to give pure 3-hydroxymethylazetidine-3-carboxylic acid (0.63 g) as white crystals in 53% yield m.p. 180° (dec.).

*Analysis*

| | | | |
|---|---|---|---|
| Calculated | C 45.75; | H 6.92; | N 10.6 |
| Found | C 45.49; | H 6.92; | N 10.58 |

Example 2

Preparation of azetidine-3,3-dicarboxylic acid

1-Tosyl-3,3-dicarboxylazetidine (N-tosylazetidine-3,3-dicarboxylic acid) (2.0 g, 0.0067 mol) (prepared as in Example 9B of European Patent Application Publication No. 114,079) was dissolved in liquid ammonia (125 ml) and the temperaturer of the resulting solution was maintained in the range −50°C to −35°C during addition of metallic sodium (1.1 g, 0.0478 g atom) in small pieces until the blue colour persisted for 15 mins. Solid ammonium chloride (2.7 g, 0.05 mol) was then added in order to disperse the blue colour and the ammonia allowed to evaporate under a stream of nitrogen. Water was added to the residue and the mixture acidified with 4 molar hydrochloric acid, whereupon the product precipitated out. Filtering and drying gave the title product (0.77 g) (85% yield) shown by 13C and proton nuclear magnetic resonance and high pressure liquid chromatographic analysis to be 99% pure. M.p. 260°C (dec.). The product was found to be soluble only in basic aqueous media.

Example 3

Preparation of azetidine-3,3-dicarboxylic acid

(i) Preparation of 3,3-bis(hydroxymethyl)azetidine)

3-bromomethyl-3-hydroxymethyl oxetane (7.5 g; 41 mmol) was taken up in 25% (w/w) aqueous ammonia (100 ml) and the mixture was stirred for 16 hours in an autoclave at 80°C. After evaporation of the aqueous ammonia, 48% (w/w) aqueous hydrobromic acid (10 ml) was added to the residue and the resulting solution was stirred at 60°C. After 2 hours the aqueous hydrobromic acid was evaporated off, 25% (w/w) aqueous ammonia (75 ml) was added to the residue, and the resulting solution was stirred at 60°C. After 2 hours the aqueous ammonia was evaporated. The white, solid residue was freed from ammonium bromide by purification over "Dowex" 50W-X8 ($H^+$) (registered Trade Mark) ion exchange resin, to yield crystalline 3,3-bis(hydroxy-methyl)azetidine (4.35 g; 90% yield on starting oxetane.

(ii) Preparation of azetidine-3,3-dicarboxylic acid

3,3-Bis(hydroxymethyl)azetidine (1.03 g) was dissolved in 55% w/w aqueous nitric acid (10 ml) and the resulting solution was stirred for 18 hours at 50°C. The temperature of the solution was then raised to 95°C and air was bubbled through. After 1 hour the solution was evaporated to yield azetidine-3,3-dicarboxylic acid as a white solid (1.15 g, 90%, m.p. 260°C dec.)) (shown to be 100% azetidine-3,3-dicarboxylic acid by proton nuclear magnetic resonance).

Example 4

Preparation of azetidine-3,3-dicarboxylic acid

3,3-Bis[hydroxymethyl]-1-methylazetidine (1.0 g) (prepared as in Example 3 but using methylamine instead of ammonia) was dissolved in 55% (w/w) aqueous nitric acid (10 ml) and the resulting mixture was stirred for 18 hours at 50°C. According to proton nuclear magnetic resonance, about 85% conversion to 1-nitroso-azetidine-3,3-dicarboxylic acid had taken place. Another 5 ml of 55% w/w aqueous nitric acid was added and the reaction was allowed to continue for a further 24 hours. According to proton nuclear magnetic resonance, conversion to 1-nitroso-azetidine-3,3-dicarboxylic acid was complete. The temperature of the reaction mixture was raised to 95°C and air was bubbled through. After 1 hour the resultant soluton was evaporated to yield the title product as a pale yellow solid (0.92 g, 83% yield), which was found by high pressure liquid chromatography to contain only trace amounts of azetidine-3-carboxylic acid.

The same product may also be obtained by application of a similar treatment to 3-hydroxymethyl azetidine-3-carboxylic acid.

Example 5

Preparation of azetidine-3-carboxylic acid

Azetidine-3,3-dicarboxylic acid (150 mg) was dispersed in 80% v/v aqueous formic acid (1 ml) at ambient temperature (20°C). The resulting slurry was heated with stirring for 6 hours under reflux (100°C), to yield a clear solution which was shown by proton nuclear magnetic resonance to contain exclusively the monocarboxylic acid derivative, azetidine-3-carboxylic acid (104.5 mg, 100%).

**Claims**

1. An azetidine derivative of formula

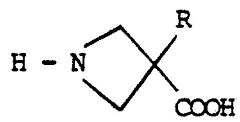

(I)

or a salt thereof, wherein R is a group $—CH_2OH$ or —COOH.

2. A process for preparing azetidine-3-carboxylic acid or a salt thereof which comprises heating a compound of formula I as defined in Claim 1 wherein R is a group —COOH, or a salt thereof in acidic medium at a temperature of at least 60°C.

3. A process according to Claim 2 wherein the compound of formula I wherein R is a group —COOH is generated by oxidation of a compound of formula I wherein R is a group $—CH_2OH$.

4. A process according to Claim 2 or 3 wherein the acid is a carboxylic acid.

5. A process according to Claim 4 wherein the carboxylic acid is employed in a concentration of at least 50% v/v.

6. A process according to any of Claims 2 to 5 wherein the compound of formula I is heated at the reflux temperature of the reaction mixture.

**Patentansprüche**

1. Azetidinderivat mit der Formel

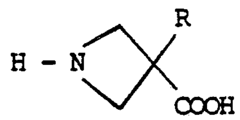

(I)

oder ein Salz hievon, worin R für eine Gruppe $—CH_2OH$ oder —COOH steht.

2. Verfahren zur Herstellung von Azetidin-3-carbonsäure oder einem Salz hievon, welches ein Erhitzen einer Verbindung der Formel I, wie in Anspruch 1 definiert, worin R für eine Gruppe —COOH steht, oder eines Salzes hievon in saurem Medium auf eine Temperatur von wenigstens 60°C umfaßt.

3. Verfahren nach Anspruch 2, worin die Verbindung der Formel I, worin R für eine Gruppe —COOH steht, durch Oxidation einer Verbindung der Formel I, worin R für eine Gruppe $—CH_2OH$ steht, gebildet wird.

4. Verfahren nach Anspruch 2 oder 3, worin die Säure eine Carbonsäure ist.

5. Verfahren nach Anspruch 4, worin die Carbonsäure in einer Konzentration von wenigstens 50% Volumen/Volumen eingesetzt wird.

6. Verfahren nach einem der Ansprüche 2 bis 5, worin die Verbindung der Formel I zur Rückflußtemperatur des Reaktionsgemisches erhitzt wird.

**Revendications**

1. Un dérivé d'azétidine de formule

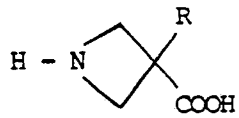

(I)

ou un sel d'un tel composé, où R est un groupe $-CH_2OH$ ou $-COOH$.

2. Un procédé de préparation de l'acide azétidine-3-carboxylique ou d'un sel de cet acide, qui comprend le chauffage d'un composé de formule I tel que défini dans la revendication 1 dans lequel R est un groupe $-COOH$ ou d'un sel de ce composé dans un milieu acide à une température d'au moins 60°C.

3. Un procédé selon la revendication 2, dans lequel le composé de formule I dans lequel R est un groupe $-COOH$ est produit par oxydation d'un composé de formule I dans lequel R est un groupe $-CH_2OH$.

4. Un procédé selon la revendication 2 ou 3, dans lequel l'acide est un acide carboxylique.

5. Un procédé selon la revendication 4, dans lequel l'acide carboxylique est utilisé à une concentration d'au moins 50% v/v.

6. Un procédé selon l'une quelconque des revendications 2 à 5, dans lequel le composé de formule I st chauffé à la température de reflux du mélange réactionnel.